# EUROPEAN PATENT SPECIFICATION

(11) **EP 1 545 546 B1**
(45) Date of publication and mention of the grant of the patent: **04.04.2007**
(21) Application number: 03795090.4
(22) Date of filing: 11.09.2003
(51) Int. Cl.: A61K 31/551, A61P 25/14, A61P 25/16, A61K 45/06

(54) **TREATMENT OF DYSKINESIA WITH 2,3-BENZODIAZEPINES**
BEHANDLUNG VON DYSKINESIE MIT 2,3-BENZODIAZEPINEN
TRAITEMENT DE LA DYSKINÉSIE AVEC DES 2,3-BENZODIAZÉPINES

(30) Priority: 13.09.2002 GB 0221248; 25.02.2003 GB 0304227
(43) Date of publication of application: 29.06.2005
(73) Proprietor: Motac Neuroscience Limited, Manchester M15 6SE (GB)
(72) Inventor: CROSSMAN, Alan, 1.124 Stopford, School of Biol. Sc, Manchester M13 9PT (GB); HILL, Michael, Motac Neurosci. Ltd., Williams Hous, Manchester M15 6SE (GB); BROTCHIE, Jonathan, Toronto Western Research Inst., Toronto, Ontario M5T 2S8 (CA)
(74) Representative: Banford, Paul Clifford
(86) International application number: PCT/GB2003/003951
(87) International publication number: WO 2004/024163

(56) References cited:
- EP-A- 0 900 568
- WO-A-02/088096
- WO-A-02/094189
- US-A- 4 322 346
- US-B1- 6 200 970
- KONITSIOTIS S ET AL: "AMPA receptor blockade improves levodopa-induced dyskinesia in MPTP monkeys" NEUROLOGY, vol. 54, no. 8, 25 April 2000 (2000-04-25), pages 1589-1595, XP008026187 ISSN: 0028-3878
- REVUTSKY E L ET AL: "Prophylaxis of meteopathological reactions in patients with ischemic heart disease and neurocirculatory dystonia" VRACHEBNOE DELO 1988 RUSSIA, no. 2, 1988, pages 7-10, XP008026189 ISSN: 0049-6804
- TARNAWA I. ET AL: "2,3-Benzodiazepine AMPA antagonists." RESTORATIVE NEUROLOGY AND NEUROSCIENCE, (1998) 13/1-2 (41-57)., XP008026184
- SÓLYOM S ET AL: "Non-competitive AMPA antagonists of 2,3-benzodiazepine type" CURRENT PHARMACEUTICAL DESIGN 2002 NETHERLANDS, vol. 8, no. 10, 2002, pages 913-939, XP008026188 ISSN: 1381-6128
- BROTCHIE J M ET AL: "THE EFFECTS OF TOFISOPAM ON THE ACTIONS INDUCED BY L - DOPA ADMINISTRATION IN THE MPTP - LESIONED NON - HUMAN PRIMATE MODEL OF PARKINSON'S DISEASE." SOCIETY FOR NEUROSCIENCE ABSTRACT VIEWER AND ITINERARY PLANNER, vol. 2002, 2002, page Abstract No. 165.18 XP008026196 32nd Annual Meeting of the Society for Neuroscience;Orlando, Florida, USA; November 02-07, 2002
- DATABASE WPI Week 200317, 27 December 2002 (2002-12-27) Derwent Publications Ltd., London, GB; AN 2003-173629 XP002266976 BELOBORODOVA ET AL.: "Method of treatment of hypertension-hyperkinetic form of dyskinesia of biliferous ways in young persons" & RU 2 195 284 C (BELOBORODOVA ET AL.), 27 December 2002 (2002-12-27)
- BEERS M. AND BERKOW R.: "The Merck Manual of Diagnosis and Therapy, 17th Edition" 1999 , MERCK RESEARCH LABORATORIES , WHITEHOUSE STATION, N.J. XP002267011 page 1462 -page 1473

## Description

The present invention relates to the treatment of basal ganglia-related movement disorders selected from dyskinesias.

Dyskinesias are abnormal involuntary movement disorders. The abnormal movements may manifest as chorea (involuntary, rapid, irregular, jerky movements that may affect the face, arms, legs, or trunk), ballism (involuntary movements similar to chorea but of a more violent and forceful nature), dystonia (sustained muscle contractions, usually producing twisting and repetitive movements or abnormal postures or positions) or athetosis (repetitive involuntary, slow, sinuous, writhing movements, which are especially severe in the hands).

Movement and other disorders due to dysfunction of the basal ganglia and related brain structures are of major socio-economic importance. Such disorders can occur as a consequence of inherited or acquired disease, idiopathic neurodegeneration or they may be iatrogenic. The spectrum of disorders is very diverse, ranging from those associated with poverty of movement (akinesia, hypokinesia, bradykinesia) and hypertonia (e.g. Parkinson's disease, some forms of dystonia) to the involuntary movement disorders (hyperkinesias or dyskinesias e.g. Huntington's disease, levodopa-induced dyskinesia, ballism, some forms of dystonia).

Knowledge of the pathophysiological mechanisms that underlie some of these disorders makes it likely that similar mechanisms mediate disorders characterised by either hyperkinesias or dyskinesias. It is to be expected, therefore, that treatments that are effective in one form of dyskinesia may be beneficial in dyskinesias of different aetiology.

One common way in which dyskinesias arise is as a side-effect of dopamine replacement therapy for parkinsonism or other basal ganglia-related movement disorders. Parkinsonism is a syndrome of symptoms characterised by slowness of movement (bradykinesia), rigidity and/or tremor. Parkinsonian symptoms are seen in a variety of conditions, most commonly in idiopathic parkinsonism (i.e. Parkinson's disease) but also following treatment of schizophrenia, exposure to toxins/drugs and head injury. In Parkinson's disease the primary pathology is degeneration of dopaminergic neurons of the substantia nigra, pars compacta.

The most widely used symptomatic treatments for parkinsonism use dopamine-replacing agents (e.g. L-DOPA and dopamine receptor agonists). These do, however, have limitations, especially following long-term treatment. Problems can include a "wearing-off" of the anti-parkinsonian efficacy of the treatment and in particular the appearance of a range of side-effects. These side-effects may manifest as dyskinesias such as chorea and dystonia. Dyskinesia can be seen either when the patient is undergoing dopamine-replacement therapy (in the case of chorea and/or dystonia) or even when off therapy (when dystonia is prevalent). Ultimately, these side-effects severely limit the usefulness of dopaminergic treatments.

Another common cause of dyskinesias is the treatment of psychosis with neuroleptic drugs - this is known as tardive dyskinesia.

Dyskinesia also occurs in many other conditions including:
- Huntington's disease
- idiopathic dystonia
- Tourette syndrome
- "off" dystonia in parkinsonism
- ballism
- senile chorea

Many attempts have been made to develop agents that will prevent the development of, and/or treat, dyskinesias although such attempts have met with limited success. There is, therefore, a need to develop ways by which dyskinesias may be treated.

US-B-6,200,970 discloses 2,3-benzodiazepines as non-competitive AMPA inhibitors, which are useful for the treatment or prevention of, *inter alia,* neurodegenerative diseases, including Parkinson's disease and Huntington's chorea.

The present invention relates to the treatment of dyskinesias using a compound with the general formula (I) 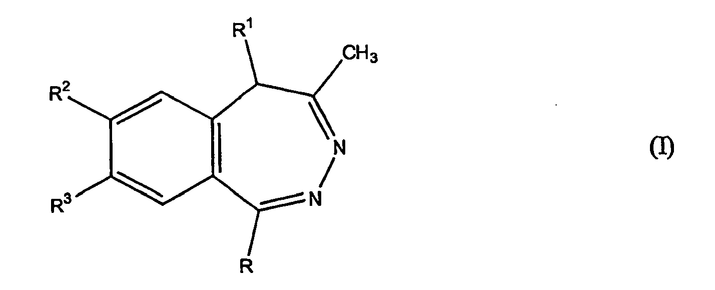 wherein R is an aryl group, such as phenyl or benzyl, which is optionally substituted with a C₁₋₆ alkyl, C₁₋₆ alkoxy, halogen, hydroxyl, amino, nitro, amido, nitrile or a carboxyl group;
R¹ is C₁₋₆ alkyl or hydrogen;
R² is C₁₋₆ alkoxy or hydroxyl and
R³ is C₁₋₆ alkoxy, hydrogen, hydroxyl, or halogen,

Preferably R is selected from one of the following groups: 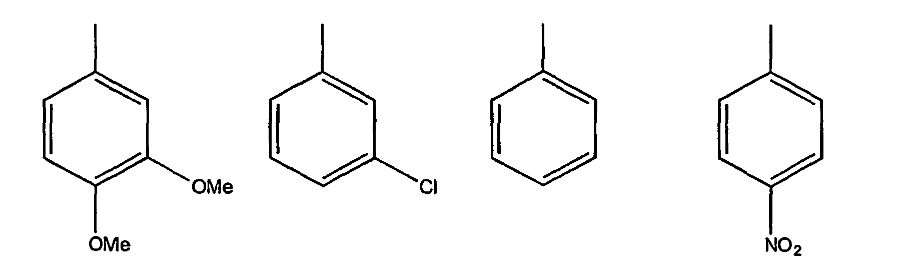 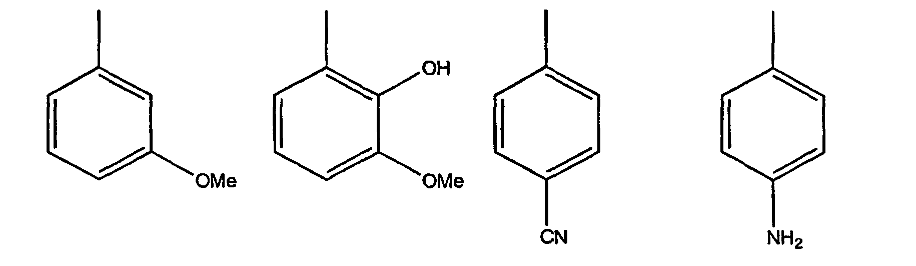 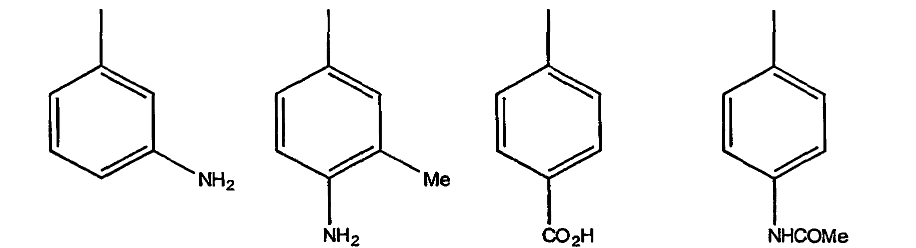 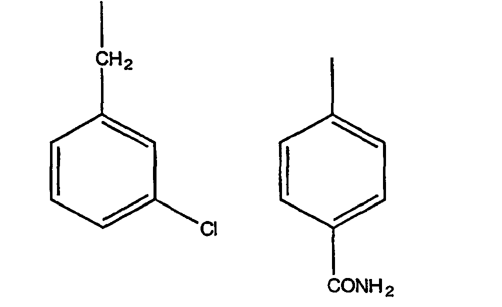
When R¹ is an alkyl group, it is preferred that R¹ is C₁₋₃ alkyl with C₂ alkyl (ethyl) being most preferred.
When R² is an alkoxy group, it is preferred that R² is C₁₋₃ alkoxy with C₁ alkoxy (methoxy) being most preferred.
When R³ is an alkoxy group, it is preferred that R³ is C₁₋₃ alkoxy with C₁ alkoxy (methoxy) being most preferred.

It is preferred that the compound of formula (I) is selected from the group of Tofisopam, Girisopam and Nerisopam, which are as follows:

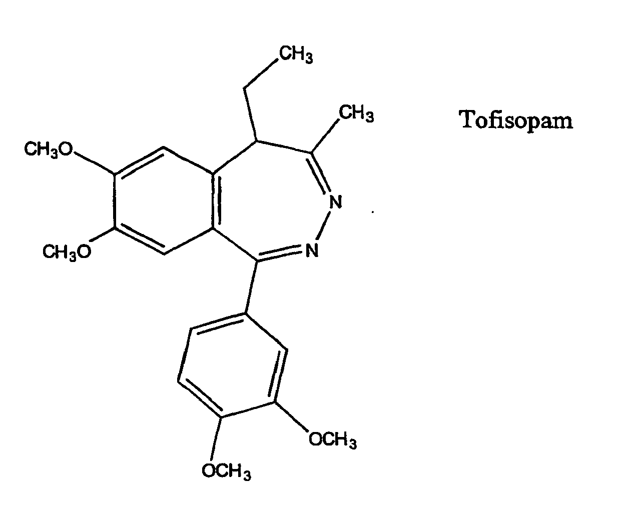

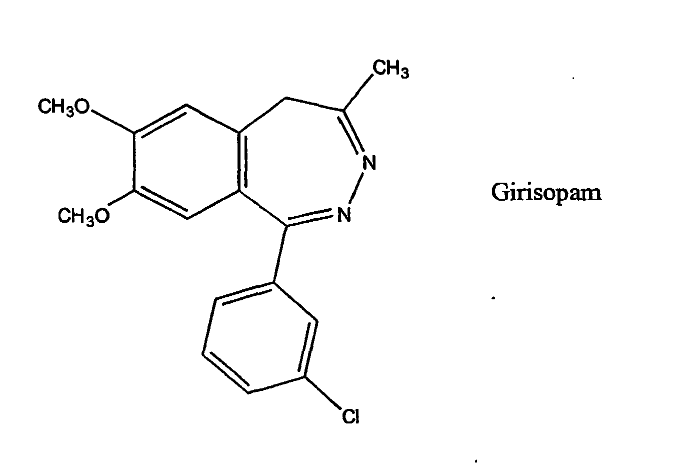

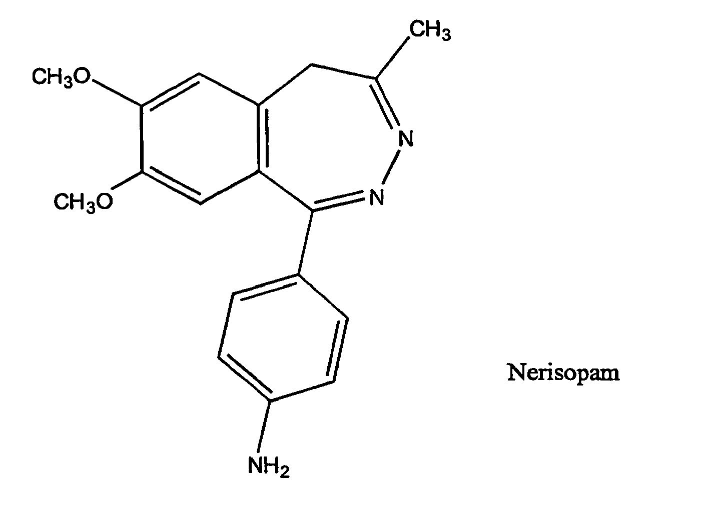

Most preferably the compound of formula (I) is Tofisopam.

It will be appreciated that different stereoisomers of compounds of formula (I) may exist and such stereoisomers may be be employed to treat dyskinesia according to the invention. For instance it will be appreciated that compounds such as Tofisopam have a chiral centre. Accordingly Tofisopam, and other compounds of formula (I) that possess a chiral centre, can exist as R and S enantiomers. The present invention encompasses the use of such compounds as a racemic mixture or the use of separated R or S enantiomers.

According to a first aspect of the present invention, there is provided a use of a compound of general formula (I) for the manufacture of a medicament for the treatment of dyskinesia.

An agent which modulates the activity of receptors with ligands of general formula (I) can be used for the manufacture of a medicament for the treatment of dyskinesia.

By "receptors" we mean receptors located on the cell bodies and terminals of neurons located within the striatum, the internal and external segments of the globus pallidus and the substantia nigra in the brain and thereby induce a neuronal signal which reduces dyskinesia and for which molecules of general formula (I) act as ligands.

By "dyskinesia" we mean abnormal involuntary movements that are associated with disorders of brain regions known as the basal ganglia. The dyskinesia may be a "levodopa-induced dyskinesia" that arises as a complication of the treatment of Parkinson's disease (the most common basal ganglia disease). Dyskinesia can physically manifest in two forms, chorea and dystonia. Chorea consists of involuntary, continuous, purposeless, abrupt, rapid, brief, unsustained and irregular movements that flow from one part of the body to another. Dystonia refers to sustained muscle contractions that cause twisting and repetitive movements or abnormal postures.

Dyskinesias may be distinguished from ataxia or catalepsy. Ataxia is usually associated with disorders of a part of the brain called the cerebellum, or its connections. It is characterised by poor motor coordination. There is a staggering gait (walk) and slurred speech, which may make the person appear "drunk". Catalepsy is, again, a different condition that is impossible to confuse with dyskinesias. It is usually associated with psychotic disorders. It is characterized by inactivity, decreased responsiveness to stimuli, and a tendency to maintain an immobile posture. The limbs tend to remain in whatever position they are placed. Thus, the terms dyskinesia (including chorea and dystonia), ataxia and catalepsy refer to distinct and separate disorders. They have different physical manifestations and different causes.

The present invention is based upon research conducted by the inventors relating to the activity of compounds of general formula I. To their surprise they found that such compounds have efficacy for reducing dyskinesias. This lead them to realise that several classes of agents may be used. These include:
(i) exogenous 2, 3 benzodiazepine receptor ligands;
(ii) compounds which enhance synthesis of endogenous 2,3 benzodiazepine receptor ligands;
(iii) compounds which enhance release of endogenous 2,3 benzodiazepine receptor ligands;
(v) compounds which block the rate of inactivation or metabolism of endogenous 2,3 benzodiazepine receptor agonists; and
(vi) compounds which promote/increase 2,3 benzodiazepine receptor expression and/or transcription.

The invention is based upon our studies relating to the neural mechanisms underlying movement disorders. Although we do not wish to be bound by any hypothesis, we believe that movement disorders involve abnormal activity of basal ganglia output pathways and in many cases this is brought about by abnormal function of striatal efferent pathways. These consist of a "direct" pathway to the medial or internal segment of the globus pallidus and the pars reticulata of the substantia nigra and a "indirect" pathway to the lateral or external segment of the globus pallidus. One of the pathophysiological hallmarks of dyskinesia is overactivity of the direct striatal output pathway. Conversely, in Parkinson's disease the direct striatal output pathway is underactive and the indirect striatal output pathway is overactive. We believe compounds of general formula (I) bind to receptors located on the terminals of the striatal output neurons that project to the internal and external segments of the globus pallidus and the pars reticulata of the substantia nigra and thereby induce a neuronal signal which reduces dyskinesia.

WO 99/06408 and WO 01/04122 disclose molecules that have a core 2, 3 benzodiazepine tricyclic structure with superficial similarity to the compounds of general formula (I). The prior art speculates that such compounds may be used in the treatment of a variety of medical conditions (e.g. Parkinson's disease). However, the compounds used according to the invention are distinguished over this prior art in that the rings have different substituents (in particular at R, R² and R³). Furthermore, the compounds of general formula (I) have surprising utility for treating dyskinesias, that is to say excessive involuntary movements, and not Parkinson's disease *per se,* which is characterised by a poverty of movement.

The present inventors conducted experiments with the molecule Tofisopam that led them to realise that compounds of general formula (1) are highly effective for the treatment of dyskinesias. For instance, it was found that dyskinesias (e.g. chorea and dystonia) do not develop, or are at least reduced, when the compounds are given to subjects on dopamine-replacement therapy for the treatment of a movement disorder.

The compounds (and compositions or medicaments containing them) may be used to treat many types of dyskinesia. For instance the compounds may be used to treat dyskinesia associated with Huntington's disease, idiopathic torsion dystonia, tardive dyskinesia or off-dystonia in Parkinson's disease and most particularly for dyskinesia associated with movement disorders such as parkinsonism (e.g. idiopathic Parkinson's disease, post-encephalitic parkinsonism or parkinsonism resulting from head injury), treatment of schizophrenia, drug intoxication, manganese poisoning and the like.

The compounds may also be used in the treatment of dyskinesias that manifest as hyperkinetic activity (e.g. Tourette's syndrome or attention deficit hyperactivity (ADHD)).

The compounds are also useful for treatment of dyskinesias that arise as a side-effect of other therapeutic agents. For instance, the compounds are useful for the treatment of dyskinesia associated with ropinirole, pramipexole, cabergoline, bromcriptine, lisuride, pergolide, L-DOPA or apomorphine treatment. The compounds are preferably used for the treatment of dyskinesia associated with L-DOPA or apomorphine treatment.

Levodopa is an aromatic amino acid. The chemical name of levodopa or L-DOPA is (-)-L-α-amino-β-(3,4-dihydroxybenzene) propanoic acid. L-DOPA has the molecular formula C₉H₁₁NO₄ and a molecular weight of 197.2. Chemically, levodopa is (-)-3-(3,4-dihydroxy-phenyl)-L-alanine. It is a colorless, crystalline compound, slightly soluble in water and insoluble in alcohol. L-DOPA has the following structural formula:

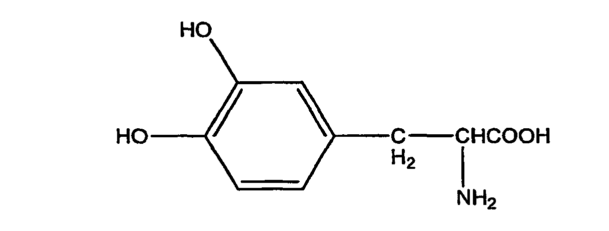

Because L-DOPA is an amino acid, it is commonly administered to patients in combination with carbidopa for the treatment of Parkinson's disease and syndrome. The chemical name for carbidopa is (-)-L-α-hydrazino-α-methyl-β-(3,4-dihydroxybenzene) propanoic acid monohydrate. Carbidopa has the empirical formula C₁₀H₁₄N₂O₄•H₂O and a molecular weight of 244.3. Anhydrous carbidopa has a molecular weight of 226.3. Sinemet^{®} is a combination of carbidopa and levodopa for the treatment of Parkinson's disease and syndrome. Sinemet^{®} is described in U.S. Patents 4,832,957 and 4,900,755. The structural formula of carbidopa is:

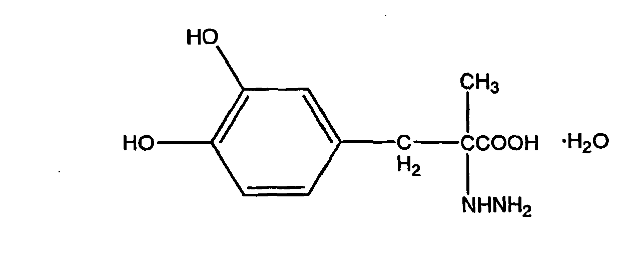

In addition, the compounds according to the invention are useful for the treatment of dyskinesias associated with ropinirole treatment. Ropinirole is a non-ergoline dopamine agonist sold under the trademark Requip^{®}. Ropinirole is the hydrochloride salt of 4-[2-(dipropylamino)ethyl]-1,3-dihydro-2H-indol-2-one monohydrochloride and has an empirical formula of C₁₆H₂₄N₂O·HCl. The molecular weight of ropinirole is 296.84 (260.38 as the free base). Ropinirole is described in U.S. Patent Numbers 4,452,808 and 4,824,860. The structural formula of ropinirole is:

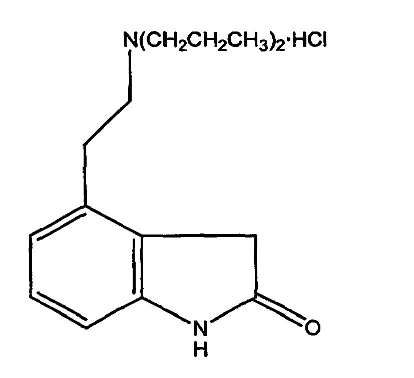

The compounds according to the invention are also useful for the treatment of dyskinesias associated with pramipexole treatment. The chemical name of pramipexole is (S)-2-amino- 4,5,6,7-tetra-hydro-6-(propylamino) benzothiazole dihydrochloride mono-hydrate. Pramipexole dihydrochloride is sold under the trademark Mirapex^{®}. Pramipexole dihydrochloride has the empirical formula C₁₀H₁₇N₃S·2HCl·H₂O and a molecular weight of 302.27. The synthesis of pramipexole is described in U.S. Patents 4,843,086 and 4,886,812. The structural formula of pramipexole dihydrochloride is:

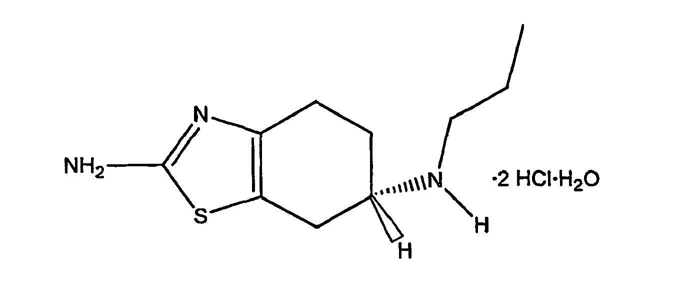

The compounds may also be used for the treatment of dyskinesias associated with cabergoline treatment. The chemical name for cabergoline is 1-adamantanamine hydrochloride. It has a molecular weight of 187.71 and a molecular formula of C₁₀H₁₈NCl. The structural formula of cabergoline is:

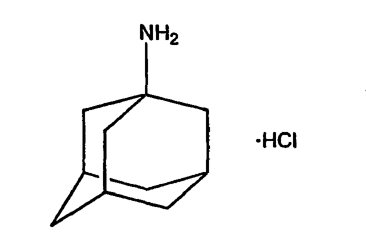

The compounds may also be used for the treatment of dyskinesias associated with bromocriptine treatment. Bromocriptine mesylate is sold under the trademark Parlodel®. The chemical name for bromocriptine mesylate is Ergotaman-3',6',18-trione, 2-bromo-12'-hydroxy 2'-(1 methylethyl)-5'-(2-methylpropyl)-, (5'a)-monomethanesulfonate. The molecular weight of bromocriptine mesylate is 750.70 and it has an empirical formula of C₃₂H₄₀BrN₅O₅· CH₄SO₃. The structural formula of bromocriptine mesylate is:

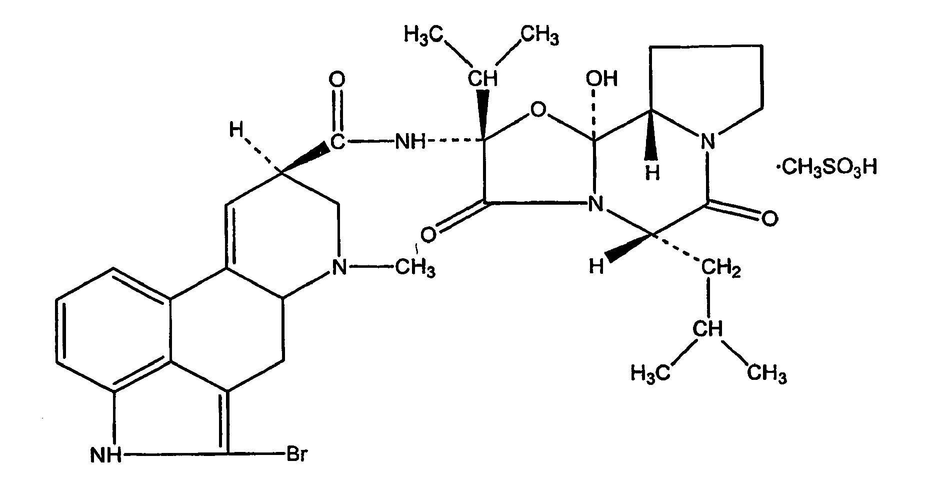

The compounds may also be used for the treatment of dyskinesias associated with lisuride treatment. The chemical name for lisuride is R(+)-N'-[(8α,)-9,10-Didehydro-6-methylergolin-8-yl]-N,N-diethylurea hydrogen maleate. Lisuride has a molecular weight of 338.45 and the empirical formula C₂₀H₂₆N₄O. The structural formula of lisuride is:

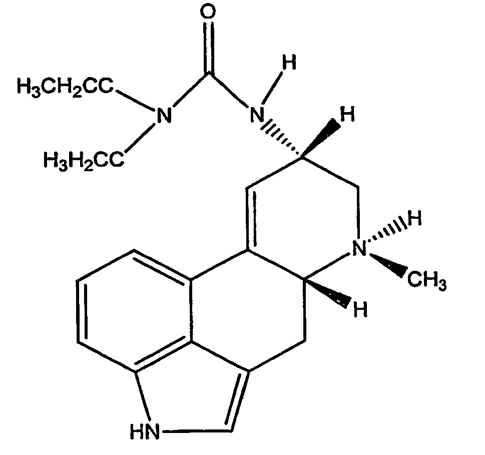

The compounds may also be used for the treatment of dyskinesias associated with pergolide treatment. The chemical name of pergolide mesylate is 8β-[(Methylthio)methyl]-6-propylergoline monomethanesulfonate. Pergolide mesylate is sold under the trademark Permax^{®}. Permax has the empirical formula C₁₉H₂₆N₂S•CH₄O₃S and a molecular weight of 410.59. The synthesis of pergolide mesylate is described in U.S. Patent Numbers 4,797,405 and 5,114,948. The structural formula of pergolide mesylate is:

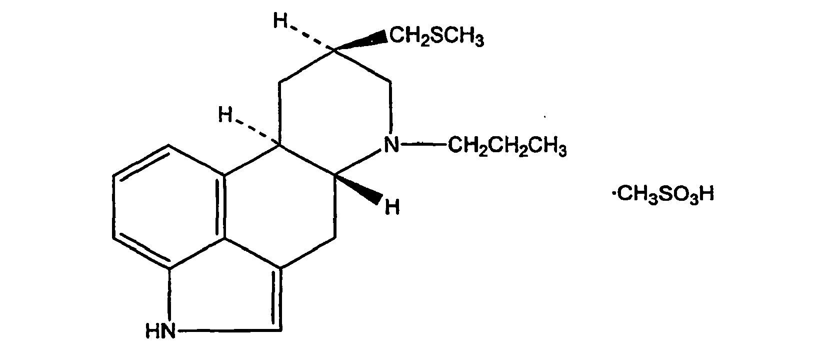

The compounds may also be used for the treatment of dyskinesias associated with apomorphine treatment. Apomorphine has the empirical formula C₁₇H₁₇NO₂ and a molecular weight of 267.33. The structural formula of apomorphine is:

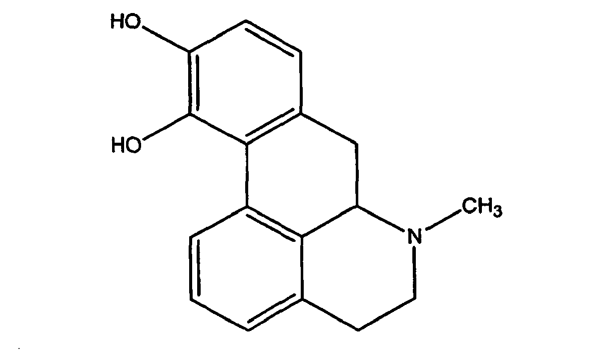

The compounds are particularly useful for treating dyskinesia caused by agents used to treat movement disorders such as parkinsonism. In this respect a preferred use of the compounds is in the treatment of dyskinetic side-effects associated with L-DOPA, apomorphine or other dopamine agonist therapy for parkinsonism.

The compounds may be used to treat existing dyskinesias but may also be used when prophylactic treatment is considered medically necessary. For instance, when it is considered necessary to initiate L-DOPA therapy and it is feared that dyskinesias may develop.

The compounds may be used to treat dyskinesia as a monotherapy (i.e. use of the compound alone) or they may be used as an adjunct to other therapeutic agents. For instance, the compounds may be co-administered with therapeutic agents to prevent dyskinetic side-effects caused by such therapeutic agents (e.g. as an adjunct to L-DOPA or apomorphine given to treat parkinsonian patients) or alternatively the compounds may be given in combination with other treatments which also reduce dyskinesia (e.g. µ-opioid receptor antagonists, α₂-adrenoreceptor-antagonists, cannabinoid CB₁-antagonists, NMDA receptor-antagonists, cholinergic receptor-antagonists, histamine H3-receptor agonists, and globus pallidus/subthalamic nucleus lesion/deep brain stimulation).

In preferred embodiments of the invention the compound of general formula (I) may be combined with therapeutic agents such as:
(a) therapeutic agents used in the treatment of parkinsonism, including Parkinson's disease (e.g. L-DOPA, Chloro-APB, apomorphine, ropinirole, pramipexole, cabergoline, bromcriptine, lisuride or pergolide)
(b) other therapeutic agents used in the treatment of dyskinesia (e.g. non selective, δ or µ-opioid receptor antagonists, α₂-adrenoreceptor-antagonists, cannabinoid CB₁-antagonists, Histamine H3 agonists, mGLuR antagonists NMDA receptor-antagonists, Gpi lesion/deep brain stimulation).
(c) therapeutic agents used as neuroleptics for the treatment of schizophrenia and psychosis (e.g. agents with dopamine receptor antagonist properties, haloperidol clozapine, fluphenazine and sulpiride).

The compounds may also be used as an adjunct or in combination with known therapies. For instance, we have found that the combination of L-DOPA with compounds according to the invention results in movement disorders such as Parkinson's disease being treated with significantly reduced dyskinetic side-effects.

The compounds may also be used in combination with a known neuroleptic to treat patients suffering from tardive dyskinesia. The term neuroleptic refers to the effects on cognition and behavior of antipsychotic drugs that reduce confusion, delusions, hallucinations, and psychomotor agitation in patients with psychoses. There is a naturally occurring chemical, a neurotransmitter, in the brain called dopamine. Dopamine is the chemical messenger in the brain mainly involved with thinking, emotions, behavior and perception. In some illnesses, dopamine may be overactive and upsets the normal balance of chemicals in the brain. This excess dopamine helps to produce some of the symptoms of the illness. The main effect that these drugs have is to block some dopamine receptors in the brain, reducing the effect of having too much dopamine and correcting the imbalance. This reduces the symptoms caused by having too much dopamine.

Neuroleptic drugs are a class of antipsychotics. Examples of neuroleptic compounds include: haloperidol (Haldol), chlorpromazine (Thorazine), thioridazine (Mellaril), risperidone (Risperdal), quetiapine (Seroquel), olanzapine (Zyprexa), clozapine (Clozaril), amisulpride (Solian), sertindole (Serdolect), zotepine (Zoleptil), Thiothixene (Navane), Molidone (Moban), Loxapine (Loxitane), Prochlorperazine (Compazine), Trifluoperazine (Stelazine), Perphenazine (Trilafon), and Metaclopramide (Reglan).

Haloperidol has a molecular formula of C₂₁H₂₃ClFNO₂ and a molecular weight of 375.8696 g/mol. Haloperidol is also referred to as Haldol; 4-[4-(p-chlorophenyl)-4-hyaroxypiperidino]-4'-fluorobutyrophenone; gamma-(4-(para-Chlorophenyl)-4-hydroxypiperidino)-para'-fluorobutyrophenone; and Serenace.

Chlorpromazine hydrochloride, a phenothiazine derivative, has a chemical formula of 2-chloro-10-[3(-dimethylamino) propyl] phenothiazine monohydrochioride. Chlorpromazine hydrochloride has the molecular formula: C₁₇H₁₉ClN₂S•HCl and a molecular weight of 355.33.

SEROQUEL® (quetiapine fumarate) is an antipsychotic drug belonging to a new chemical class, the dibenzothiazepine derivatives. The chemical designation of quetiapine fumarate is 2-[2-(4-dibenzo [b,f] [1,4]thiazepin-11-yl-1-piperazinyl)ethoxy]-ethanol fumarate (2:1) (salt). Quetiapine fumarate is present in tablets as the fumarate salt. All doses and tablet strengths are expressed as milligrams of base, not as fumarate salt. Quetiapine fumarate has a molecular formula of C₄₂H₅₀N₆O₄S₂•C₄H₄O₄ and a molecular weight of 883.11 (fumarate salt).

The chemical name for clozapine is 8-chloro-11-(4-methyl-1-piperazinyl)-5H-dibenzo [b,e] [1,4] diazepine. Clozapine is a an atypical antipsychotic drug which is a tricyclic dibenzodiazepine derivative. Clozapine is sold under the trademark "CLOZARIL®". Clozapine has a molecular weight of 326.83 and a molecular formula of C₁₈H₁₉ClN₄.

The chemical name of trifluoperazine hydrochloride is 10-[3-(4-methyl-1-piperazinyl) propyl]-2-(trifluoromethyl) phenothiazine dihydrochloride. Trifluoperazine has a molecular weight of 480.43 and a molecular formula of C₂₁H₂₄F₃N₃S•2HCl.

Metoclopramide hydrochloride is a white crystalline, odorless substance, freely soluble in water. The chemical name of metoclopramide is 4-amino-5-chloro-N-[2-(diethylamino)ethyl]-2-methoxy benzamide monohydrochloride monohydrate. Metoclopramide has a molecular weight of 354.3.

Fluphenazine hydrochloride is a trifluoro-methyl phenothiazine derivative intended for the management of schizophrenia. The chemical name of fluphenazine is 4-[3-[2-(Trifluoro-methyl) phenothiazin-10-yl] propyl]-1-piperazineethanol dihydrochloride.

The molecular formular of fluphenazine is C₂₂H₂₆F₃N₃OS•2HCl and its molecular weight is 510.44.

Compositions according to the first, second, third or fourth aspects of the invention may take a number of different forms depending, in particular on the manner in which the composition is to be used. Thus, for example, the composition may be in the form of a powder, tablet, capsule, liquid, ointment, cream, gel, hydrogel, aerosol, spray, micelle, transdermal patch, liposome or any other suitable form that may be administered to a person or animal. It will be appreciated that the vehicle of the composition of the invention should be one which is well tolerated by the subject to whom it is given and enables delivery of the compounds to the brain.

The compositions may be used in a number of ways. For instance, systemic administration may be required in which case the compound may be contained within a composition which may, for example, be ingested orally in the form of a tablet, capsule or liquid. Alternatively, the composition may be administered by injection into the blood stream. Injections may be intravenous (bolus or infusion) or subcutaneous (bolus or infusion). The compounds may also be administered by inhalation (e.g. intranasally).

Compounds as defined by general formula (I) may also be administered centrally by means of intracerebral, intracerebroventricular, or intrathecal delivery.

The compositions are particularly useful when incorporated into patches that may be applied to the skin for transdermal delivery of the compounds according to general formula (I).

The compound may also be incorporated within a slow or delayed release device. Such devices may, for example, be inserted on or under the skin and the compound may be released over weeks or even months. Such a device may be particularly useful for patients with long-term dyskinesia such as patients on continuous L-DOPA therapy for the treatment of Parkinsonism. The devices may be particularly advantageous when a compound is used which would normally require frequent administration (e.g. at least daily ingestion of a tablet or daily injection).

It will be appreciated that the amount of a compound required is determined by biological activity and bioavailability which in turn depends on the mode of administration, the physicochemical properties of the compound employed and whether the compound is being used as a monotherapy or in a combined therapy. The frequency of administration will also be influenced by the abovementioned factors and particularly the half-life of the compound within the subject being treated.

Optimal dosages to be administered may be determined by those skilled in the art, and will vary with the particular compound in use, the strength of the preparation, the mode of administration, and the advancement of the disease condition. Additional factors depending on the particular subject being treated will result in a need to adjust dosages, including subject age, weight, gender, diet, and time of administration.

Known procedures, such as those conventionally employed by the pharmaceutical industry (e.g. *in vivo* experimentation, clinical trials etc), may be used to establish specific formulations of compositions and precise therapeutic regimes (such as daily doses of the compounds and the frequency of administration).

Generally, a daily dose of between 0.01 µg/kg of body weight and 1.0g/kg of body weight of a compound of general formula (I) may be used for the treatment of dyskinesia depending upon which specific compound is used, more preferably, the daily dose is between 0.01mg/kg of body weight and 100mg/kg of body weight.

Purely by way of example a suitable dose of tofisopam for treating L-DOPA induced dyskinesia in subjects with Parkinson's disease is between 0.1mg/kg/day and 100mg/kg/day (depending upon the health status of the individual). It is preferred that between 0.25mg/kg/day and 20mg/kg/day of tofisopam is given to a person daily and is most preferred that about 10mg/kg/day or 15 mg/kg/day tofisopam is given for treating dyskinesia induced by L-DOPA.

It will be appreciated that the required dose will be influenced by the route of administration. When tofisopam is given intravenously 0.1-10 mg/kg is a preferred dose whereas higher doses (e.g. 5-15 mg/kg) may be a suitable dose orally.

By way of further example suitable doses of Girisopam or Nerisopam are preferably 0.5-30 mg/kg.

Daily doses may be given as a single administration (e.g. a daily tablet for oral consumption or as a single daily injection). Alternatively the compound used may require administration twice or more times during a day. As an example, tofisopam for treating L-DOPA induced dyskinesia in patients with Parkinson's disease may be administered as two (or more depending upon the severity of the dyskinesia) daily doses of between 25mg and 5000mg in tablet form. A patient receiving treatment may take a first dose upon waking and then a second dose in the evening (if on a two dose regime) or at 3 or 4 hourly intervals thereafter. Alternatively a slow release device may be used to provide optimal doses to a patient without the need to administer repeated doses.

A therapeutically effective amount of the compound of the invention and a pharmaceutically acceptable vehicle can be incorporated in a pharmaceutical composition. In one embodiment, the amount of the compound (e.g. tofisopam) is an amount from 0.01 mg to 800 mg. In another embodiment, the amount is from 0.01 mg to 500 mg. When the compound is tofisopam, the amount of tofisopam may be an amount from 0.01 mg to 250 mg; preferably 0.1 mg to 60 mg; and more preferably 1 mg to 20 mg.

In an embodiment, the vehicle is a liquid and the composition is a solution. In another embodiment, the vehicle is a solid and the composition is a tablet. In a further embodiment, the vehicle is a gel and the composition is a suppository.

This invention provides the use according to claim 1 of a pharmaceutical composition made by combining a therapeutically effective amount of a compound of general formula I and a pharmaceutically acceptable vehicle.

Compounds of general formula I are preferably combined with a pharmaceutically acceptable vehicle prior to administration.

In the subject invention a "therapeutically effective amount" is any amount of a compound or composition which, when administered to a subject suffering from a disease against which the compounds are effective, causes reduction, remission, or regression of the disease. A "subject" is a vertebrate, mammal, domestic animal or human being.

In the practice of this invention the "pharmaceutically acceptable vehicle" is any physiological vehicle known to those of ordinary skill in the art useful in formulating pharmaceutical compositions.

In one embodiment, the pharmaceutical vehicle may be a liquid and the pharmaceutical composition would be in the form of a solution. In another embodiment, the pharmaceutically acceptable vehicle is a solid and the composition is in the form of a powder or tablet. In a further embodiment, the pharmaceutical vehicle is a gel and the composition is in the form of a suppository or cream. In a further embodiment the compound or composition may be formulated as a part of a pharmaceutically acceptable transdermal patch.

A solid vehicle can include one or more substances which may also act as flavoring agents, lubricants, solubilizers, suspending agents, fillers, glidants, compression aids, binders or tablet-disintegrating agents; it can also be an encapsulating material. In powders, the vehicle is a finely divided solid which is in admixture with the finely divided active ingredient. In tablets, the active ingredient is mixed with a vehicle having the necessary compression properties in suitable proportions and compacted in the shape and size desired. The powders and tablets preferably contain up to 99% of the active ingredient. Suitable solid vehicles include, for example, calcium phosphate, magnesium stearate, talc, sugars, lactose, dextrin, starch, gelatin, cellulose, polyvinylpyrrolidine, low melting waxes and ion exchange resins.

Liquid vehicles are used in preparing solutions, suspensions, emulsions, syrups, elixirs and pressurized compositions. The active ingredient can be dissolved or suspended in a pharmaceutically acceptable liquid vehicle such as water, an organic solvent, a mixture of both or pharmaceutically acceptable oils or fats. The liquid vehicle can contain other suitable pharmaceutical additives such as solubilizers, emulsifiers, buffers, preservatives, sweeteners, flavoring agents, suspending agents, thickening agents, colors, viscosity regulators, stabilizers or osmo-regulators. Suitable examples of liquid vehicles for oral and parenteral administration include water (partially containing additives as above, e.g. cellulose derivatives, preferably sodium carboxymethyl cellulose solution), alcohols (including monohydric alcohols and polyhydric alcohols, e.g. glycols) and their derivatives, and oils (e.g. fractionated coconut oil and arachis oil). For parenteral administration, the vehicle can also be an oily ester such as ethyl oleate and isopropyl myristate. Sterile liquid vehicles are useful in sterile liquid form compositions for parenteral administration. The liquid vehicle for pressurized compositions can be halogenated hydrocarbon or other pharmaceutically acceptable propellent.

Liquid pharmaceutical compositions which are sterile solutions or suspensions can be utilized by for example, intramuscular, intrathecal, epidural, intraperitoneal or subcutaneous injection. Sterile solutions can also be administered intravenously. The compounds may be prepared as a sterile solid composition which may be dissolved or suspended at the time of administration using sterile water, saline, or other appropriate sterile injectable medium. Vehicles are intended to include necessary and inert binders, suspending agents, lubricants, flavorants, sweeteners, preservatives, dyes, and coatings.

The compounds of general formula I can be administered orally in the form of a sterile solution or suspension containing other solutes or suspending agents (for example, enough saline or glucose to make the solution isotonic), bile salts, acacia, gelatin, sorbitan monoleate or polysorbate 80 (oleate esters of sorbitol and its anhydrides copolymerized with ethylene oxide).

A compound of general formula I can also be administered orally either in liquid or solid composition form. Compositions suitable for oral administration include solid forms, such as pills, capsules, granules, tablets, and powders, and liquid forms, such as solutions, syrups, elixirs, and suspensions. Forms useful for parenteral administration include sterile solutions, emulsions, and suspensions.

The compounds may be combined with a pharmaceutically acceptable vehicle and another therapeutically active agent prior to administration. The other therapeutically active agent may be for the treatment of parkinsonism (including Parkinson's disease).

In another embodiment of the present invention, the compound may combined with a pharmaceutically acceptable vehicle and another therapeutically active agent, wherein such agent is an antipsychotic agent used for the treatment of psychoses, prior to administration.

An embodiment of the present invention will now be described, by way of example, with reference to the accompanying drawings, in which;
Figure 1 is a graph illustrating the effect of Tofisopam on motor activity induced by L-DOPA in parkinsonian (MPTP-lesioned) marmosets in Example 1;
Figure 2 is a bar chart illustrating the dose/response relationship of the effect of Tofisopam on motor activity induced by L-DOPA, cumulated over a 4hour observation period, in parkinsonian (MPTP-lesioned) marmosets of Example 1;
Figure 3 is a series of bar charts (A - D) illustrating the dose/response relationship of the effect of Tofisopam on activity induced by L-DOPA, broken down into 1 hour time bins, in parkinsonian (MPTP-lesioned) marmosets of Example 1;
Figure 4 is a graph illustrating the effect of tofisopam (10 - 20mg/kg) on L-DOPA induced dyskinesia (A) and parkinsonian disability (B) in parkinsonian (MPTP-lesioned) marmosets of Example 1; and
Figure 5 is a graph illustrating the effect of tofisopam (5 - 20mg/kg) on L-DOPA induced dyskinesia (A) and parkinsonian disability (B) in parkinsonian (MPTP-lesioned) marmosets of Example 1.

For all figures: * indicates P < 0.05; ** indicates P < 0.01; and*** indicates P < 0.001 compared to L-DOPA + vehicle; non-parametric one-way repeated measures ANOVA (Friedman test) followed by Dunn's multiple comparison test.

### EXAMPLE 1

The effect of Tofisopam on L-DOPA-induced dyskinesia was assessed in the MPTP-lesioned marmoset model of Parkinson's disease.

### 1.1. Methods

### 1.1.1 Preparation of MPTP-lesioned marmoset model of Parkinson's disease

Marmosets (Callithrix jacchus) (bred in a closed colony at the University of Manchester) were rendered parkinsonian by subcutaneous injection of 2mg kg⁻¹ MPTP for 5 consecutive days. The marmosets were allowed to recover for a minimum of 10 weeks until their parkinsonism became stable. The degree of activity and disability before and after MPTP treatment was assessed using a combination of scales as described in section 1.1.2. Animals were then treated with L-DOPA for at least 3 weeks to prime them to elicit dyskinesia.

### 1.1.2 Assessment of behaviour

Behaviour was assessed using the following scales:
**(a) Activity -** a measure of the motor activity of the animals that is assessed by passive infra-red sensors every five minutes. This measure assesses all movements of the animal including dyskinesia.
**(b) Parkinsonian disability -** non-parametric measures based on the following scales:
   **Mobility score:** 0 = no movement, 1 = movement of head on the floor of the cage, 2 = movement of limbs, but no locomotion, on the floor of the cage, 3 = movement of head or trunk on wall of cage or perch, 4 = movement of limbs, but no locomotion, on wall of cage or perch, 5 = walking around floor of cage or eating from hopper on floor, 6 = hopping on floor of cage, 7 = climbing onto wall of cage or perch, 8 = climbing up and down the walls of the cage or along perch, 9 = running, jumping, climbing between cage walls / perch / roof, uses limbs through a wide range of motion and activity.
**(c) Dyskinesia -** non-parametric measures based on the following scale:
   **Dyskinesia** score: 0 = Absent, 1 = Mild, fleeting, 2 = Moderate, not interfering with normal activity, 3 = Marked, at times interfering with normal activity, 4 = Severe, continuous, replacing normal activity.

The behavioural tests were assessed every 30 minutes for 4 hours, by post hoc analysis of video-recordings by an observer blinded to the treatment

### 1.1.3 Treatments

Marmosets received all treatments as described in Table 1. The treatments were randomised such that on each day all marmosets received one of the treatments. There was at least 48 hours washout between treatments.

**Table 1**

| **Treatment Number** | **Treatment** | **Route of administration** |
|---|---|---|
| 1 | vehicle | oral |
| 2 | L-DOPA (15-17.5mg/kg) | oral |
| 3 | L-DOPA (15-17.5mg/kg) + Tofisopam (5mg/kg) | oral |
| 4 | L-DOPA (15-17.5mg/kg) + Tofisopam (10mg/kg) | oral |
| 5 | L-DOPA (15-17.5mg/kg) + Tofisopam (15mg/kg) | oral |
| 6 | L-DOPA (15-17.5mg/kg) + Tofisopam (20mg/kg) | oral |

### 1.2. Results

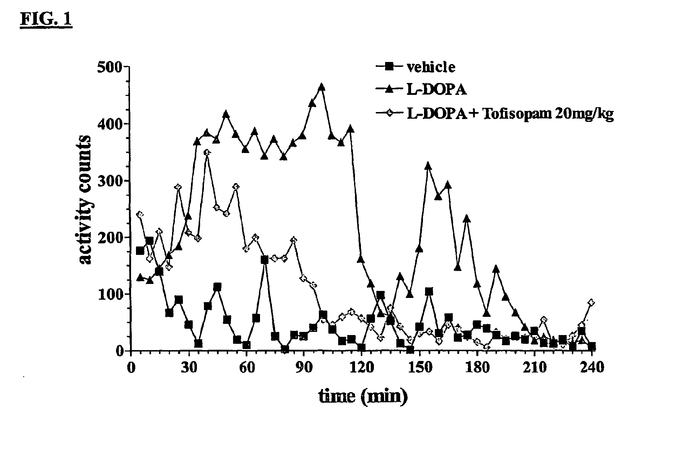

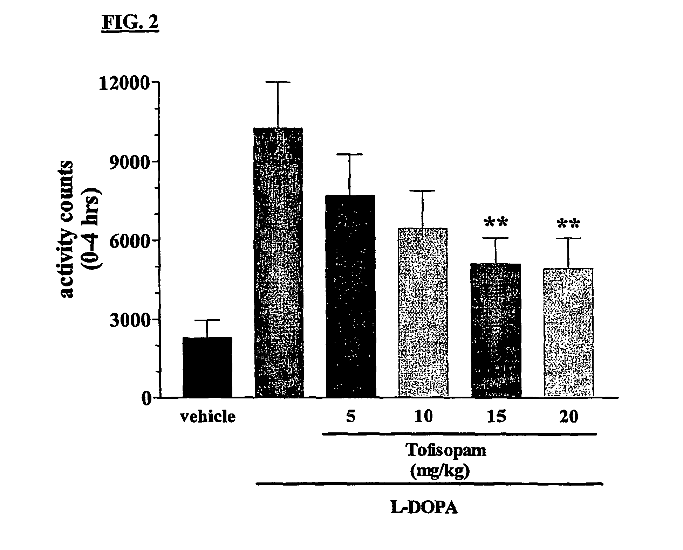

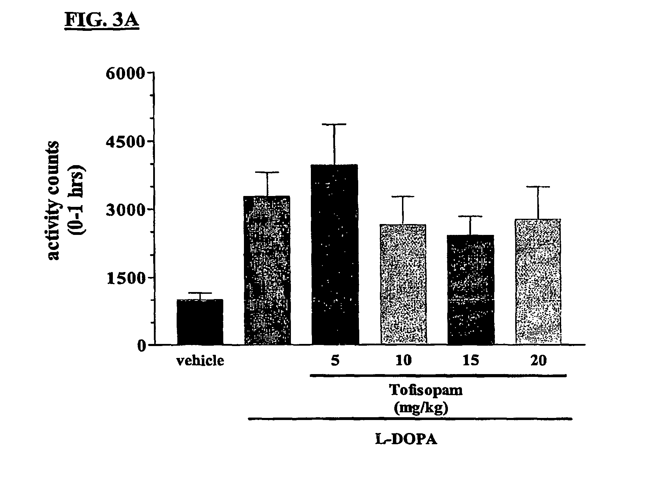

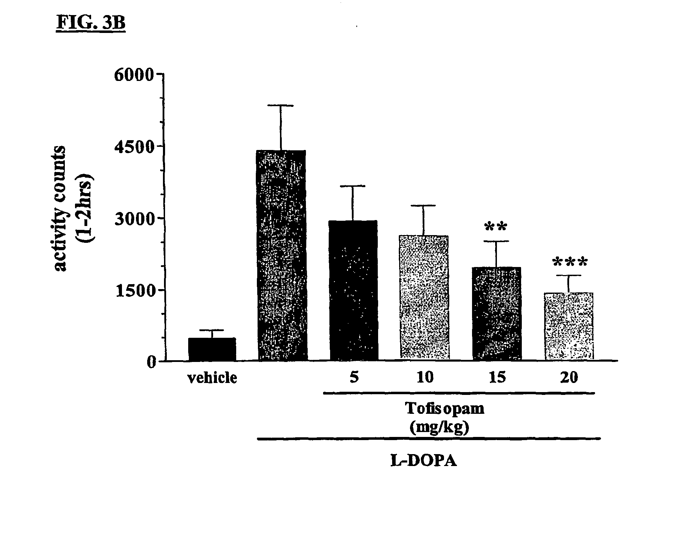

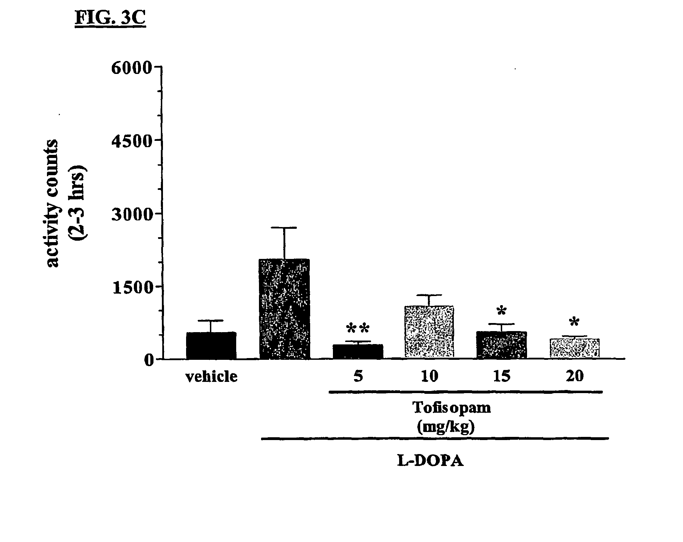

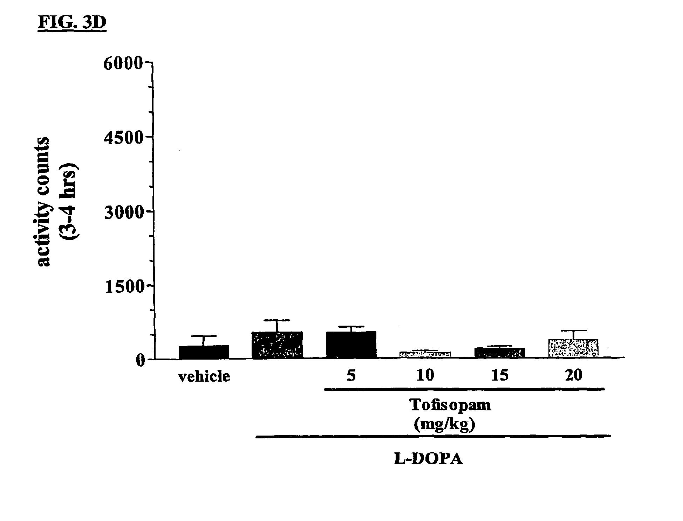

Figures 1, 2 and 3 illustrate the effect of Tofisopam treatment on L-DOPA-induced motor counts in the MPTP-lesioned marmoset model of Parkinson's disease (see method 1.1.2a).

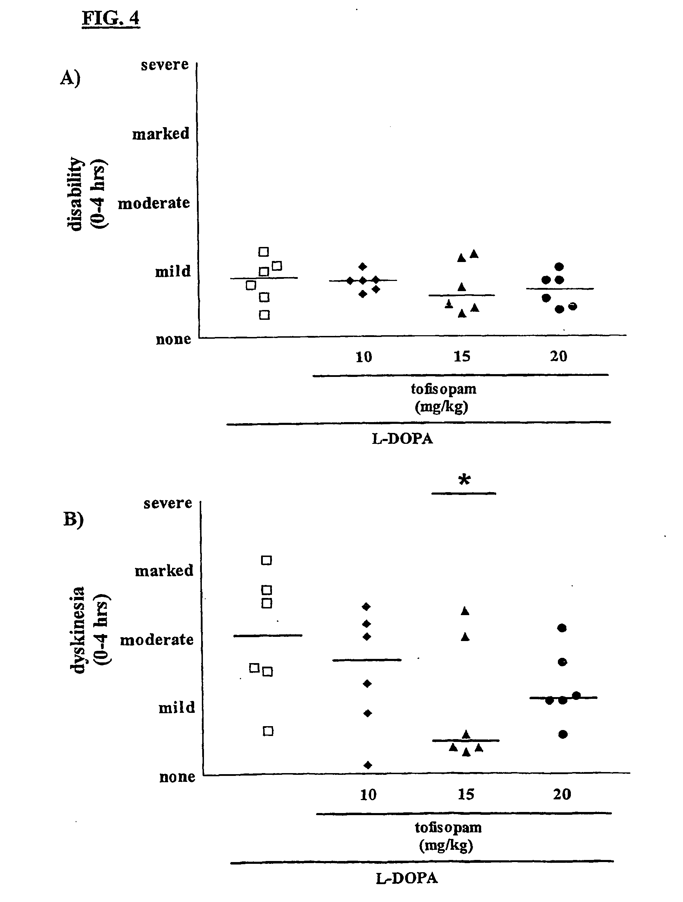

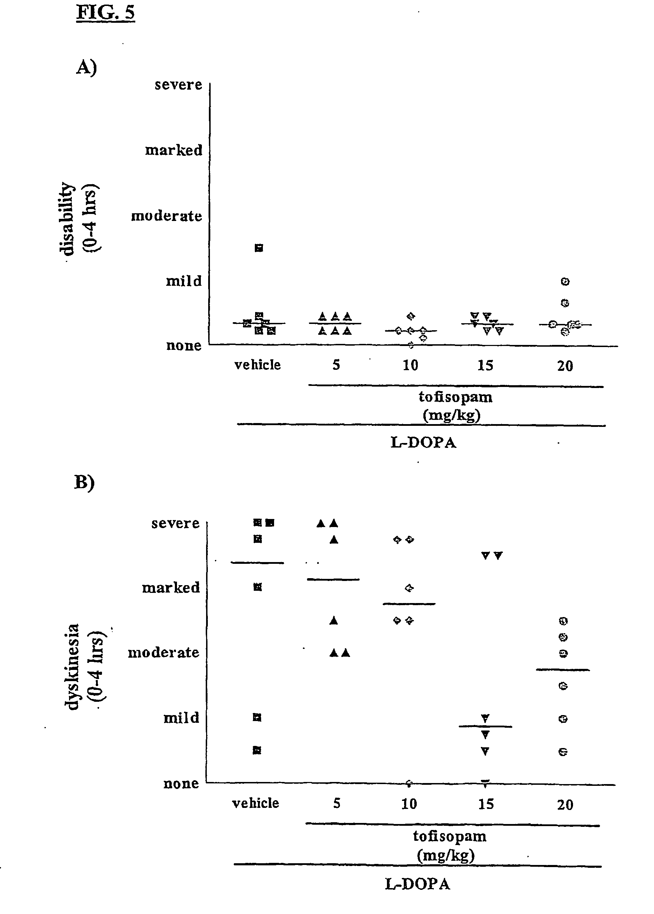

Figures 4 and 5 illustrate the results of two experiments. The figures demonstrate that Tofisopam (5 - 20 mg/kg) reduces the severity of L-DOPA-induced dyskinesia (see method 1.1.2c) without affecting the antiparkinsonian action (see method 1.1.2b) of L-DOPA.

These data demonstrate that compound as defined by general formula (I) cause a dose-dependent reduction in the severity of L-DOPA-induced dyskinesia. Figures 4 and 5, in particular, illustrate that the compounds have the benefit of reducing dyskinesia without adversely affecting the antiparkinsonian action of L-DOPA. Accordingly compounds according to the invention are particularly useful for modulating dyskinesia and, unlike prior art benzodiazepine compounds, which have been suggested to be useful for treating parkinsonism *per se.*

The MPTP-lesioned primate is the 'gold standard' preclinical model of Parkinson's disease. Therefore, these data are highly predictive of a beneficial therapeutic effect of the compounds in the treatment of L-DOPA-induced dyskinesia in Parkinson's disease patients and other dyskinesias.

## Claims

1. The use of a compound of general formula (I) 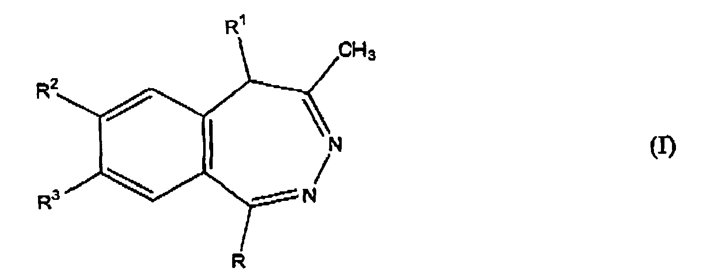 wherein
R is an aryl group selected from phenyl or benzyl, which is optionally substituted with a C₁₋₆ alkyl, C₁₋₆ alkoxy, halogen, hydroxyl, amino, nitro, amido, nitrile or a carboxyl group;
R¹ is C₁₋₆ alkyl or hydrogen;
R² is C₁₋₆ alkoxy, or hydroxyl; and
R³ is C₁₋₆ alkoxy, hydrogen, hydroxyl, or halogen,
for the manufacture of a medicament for the treatment of dyskinesia.

2. The use according to claim 1, wherein R is selected from the following groups:

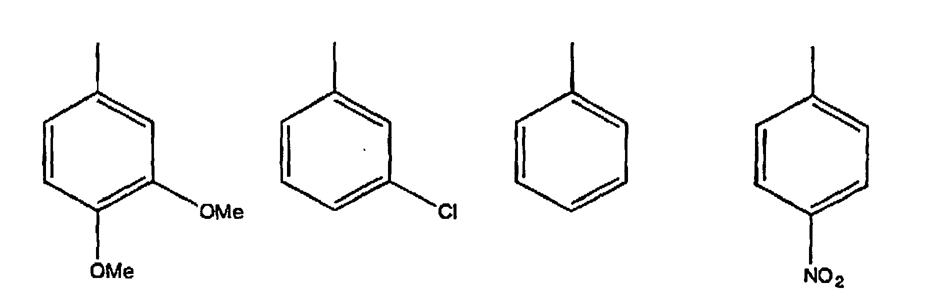

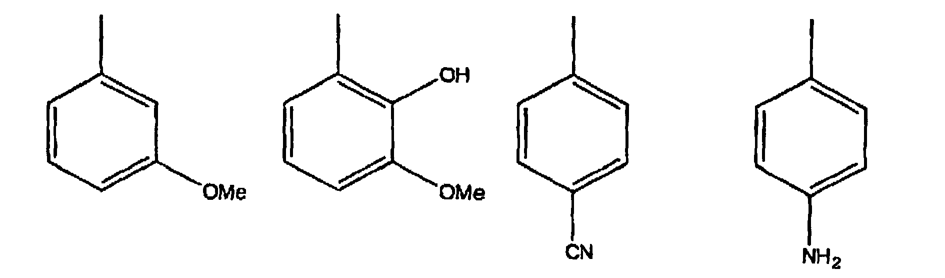

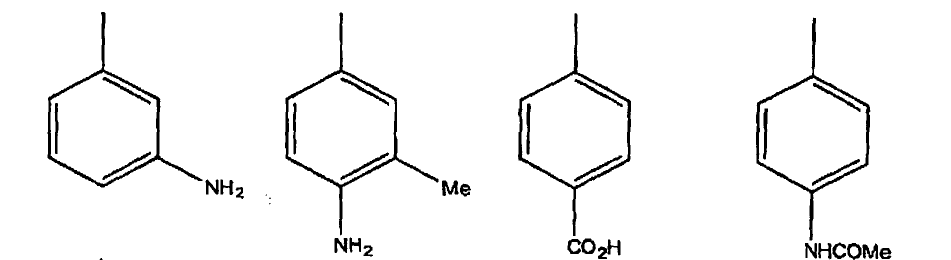

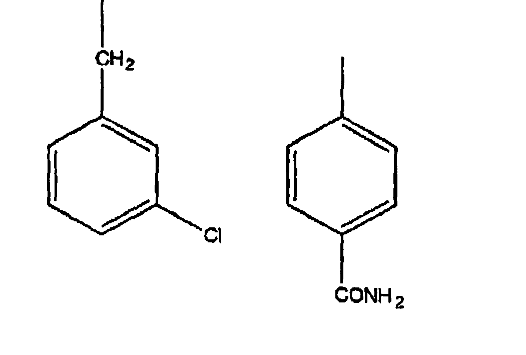

3. The use according to claim 1 or 2, wherein when R¹ is an alkyl group it is C₂ alkyl (ethyl).

4. The use according to claim 1, 2 or 3, wherein when R² is an alkoxy group, it is C₁ alkoxy (methoxy).

5. The use according to any one of the preceding claims, wherein when R³ is an alkoxy group, it is C₁ alkoxy (methoxy).

6. The use according to any one of the preceding claims, wherein the compound of formula I is selected from the group comprising Tofisopam, Girisopam and Nerisopam (shown below):

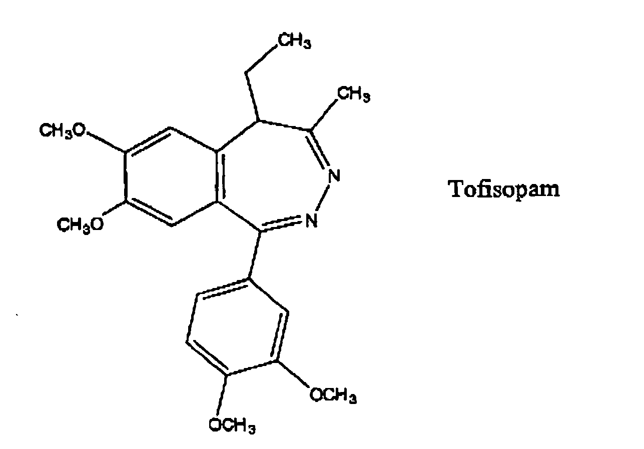

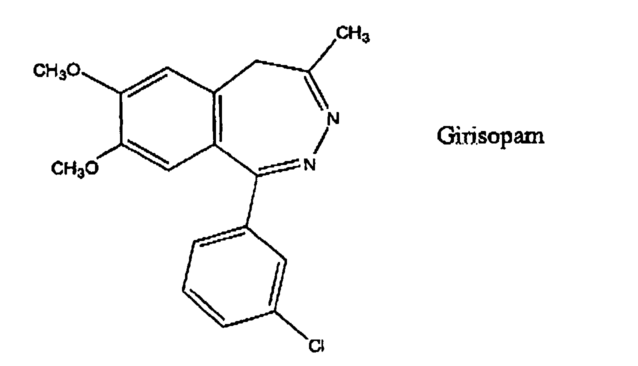

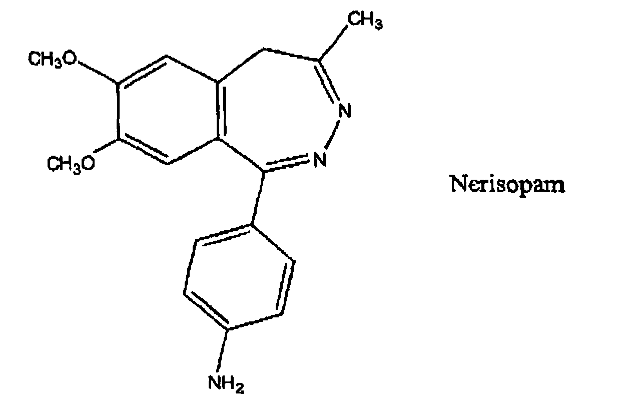

7. The use according to claim 6, wherein the compound of formula I is Tofisopam.

8. The use according to any one of claims 1 - 7 for the treatment of dyskinesia which arises as a side-effect of a therapeutic agent.

9. The use according to claim 8 for the treatment of dyskinesia associated with agents used to treat movement disorders.

10. The use according to claim 8 or 9 wherein the agent is used to treat parkinsonism.

11. The use according to claim 10 wherein the agent is a dopamine precursor.

12. The use according to claim 10 wherein the agent is a dopamine receptor agonist.

13. The use according to claim 10 wherein the agent is L-DOPA.

14. The use according to claim 10 wherein the agent is one of Chloro-APB, apomorphine, ropinirole, pramipexole, cabergoline, bromcriptine, lisuride or pergolide.

15. The use according to claim 8 wherein the agent is used to treat schizophrenia or other psychosis.

16. The use according to claim 15 wherein the agent is a neuroleptic.

17. The use according to claim 15 wherein the agent has dopamine receptor antagonist properties.

18. The use according to claim 15 wherein the agent is haloperidol, clozapine, fluphenazine or sulpiride.

19. The use according to anyone of claims 1-7 for the treatment of hyperkinetic disorder associated with Tourette's syndrome and ADHD.

20. The use according to any preceding claim for prophylactic treatment.

## Patentansprüche

1. Verwendung einer Verbindung der allgemeinen Formel (I) 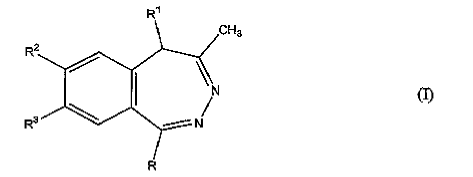 wobei
R eine Arylgruppe, ausgewählt aus Phenyl oder Benzyl, ist, welche gegebenenfalls mit einer C₁₋₆-Alkyl-, C₁₋₆-Alkoxy-, Halogen-, Hydroxyl-, Amino-, Nitro-, Amido-, Nitril- oder einer Carboxylgruppe substituiert ist;
R¹ C₁₋₆-Alkyl oder Wasserstoff ist;
R² C₁₋₆-Alkoxy oder Hydroxyl ist; und
R³ C₁₋₆-Alkoxy, Wasserstoff, Hydroxyl oder Halogen ist, für die Herstellung eines Medikaments für die Behandlung von Dyskinesie.

2. Verwendung nach Anspruch 1, wobei R aus den folgenden Gruppen: 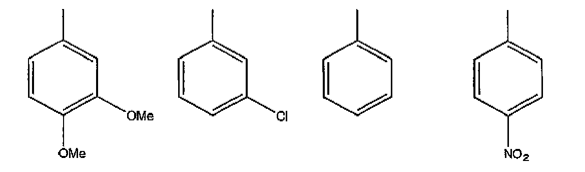 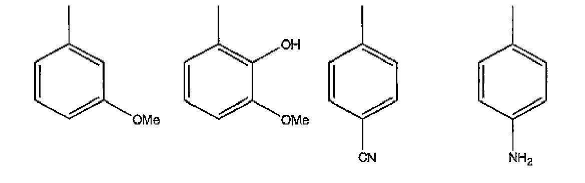 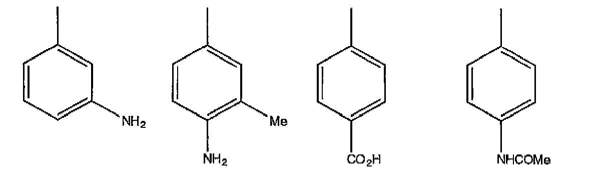 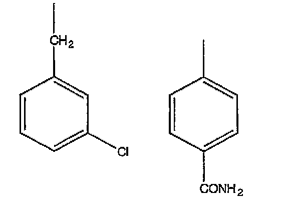 ausgewählt ist.

3. Verwendung nach Anspruch 1 oder 2, wobei, wenn R¹ eine Alkylgruppe ist, sie C₂-Alkyl (Ethyl) ist.

4. Verwendung nach Anspruch 1, 2 oder 3, wobei, wenn R² eine Alkoxygruppe ist, sie C₁-Alkoxy (Methoxy) ist.

5. Verwendung nach einem der vorhergehenden Ansprüche, wobei, wenn R³ eine Alkoxygruppe ist, sie C₁-Alkoxy (Methoxy) ist.

6. Verwendung nach einem der vorhergehenden Ansprüche, wobei die Verbindung der Formel I aus der Gruppe, umfassend Tofisopam, Girisopam und Nerisopam (nachstehend angegeben): 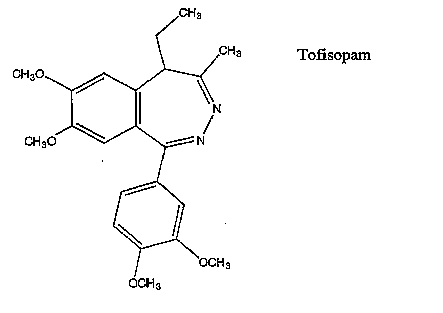 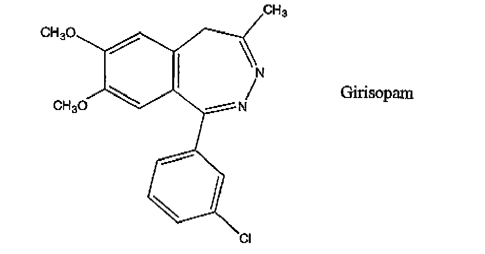 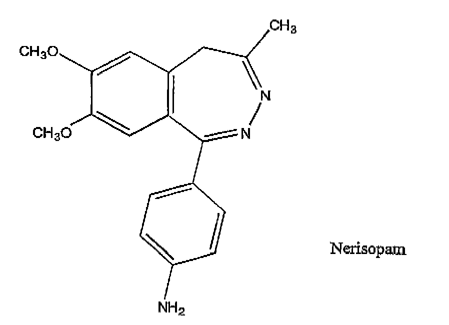 ausgewählt ist.

7. Verwendung nach Anspruch 6, wobei die Verbindung der Formel I Tofisopam ist.

8. Verwendung nach einem der Ansprüche 1 - 7 für die Behandlung von Dyskinesie, welche als Nebenwirkung eines therapeutischen Mittels entsteht.

9. Verwendung nach Anspruch 8 für die Behandlung von Dyskinesie, verbunden mit zur Behandlung von Bewegungsstörungen verwendeten Mitteln.

10. Verwendung nach Anspruch 8 oder 9, wobei das Mittel verwendet wird, um Parkinsonismus zu behandeln.

11. Verwendung nach Anspruch 10, wobei das Mittel ein Dopamin-Vorläufer ist.

12. Verwendung nach Anspruch 10, wobei das Mittel ein Dopamin-Rezeptor-Agonist ist.

13. Verwendung nach Anspruch 10, wobei das Mittel L-DOPA ist.

14. Verwendung nach Anspruch 10, wobei das Mittel eines von Chlor-APB, Apomorphin, Ropinirol, Pramipexol, Cabergolin, Bromcriptin, Lisurid oder Pergolid ist.

15. Verwendung nach Anspruch 8, wobei das Mittel verwendet wird, um Schizophrenie oder andere Psychose zu behandeln.

16. Verwendung nach Anspruch 15, wobei das Mittel ein Neuroleptikum ist.

17. Verwendung nach Anspruch 15, wobei das Mittel Dopamin-Rezeptor-Antagonist-Eigenschaften hat.

18. Verwendung nach Anspruch 15, wobei das Mittel Haloperidol, Clozapin, Fluphenazin oder Sulpirid ist.

19. Verwendung nach einem der Ansprüche 1-7 für die Behandlung von hyperkinetischer Störung, verbunden mit Tourette-Syndrom und ADHD.

20. Verwendung nach einem vorhergehenden Anspruch für prophylaktische Behandlung.

## Revendications

1. Utilisation d'un composé de la formule générale (I) 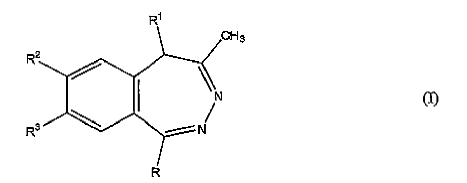 dans lequel
R est un groupe aryle sélectionné parmi phényle ou benzyle, qui est optionnellement substitué par un groupe alkyle C₁₋₆, alcoxy C₁₋₆, halogène, hydroxyle, amino, nitro, amido, nitrile ou carboxyle;
R¹ est un alkyle C₁₋₆ ou un hydrogène;
R² est un alcoxy C₁₋₆ ou un hydroxyle; et
R³ est un alcoxy C₁₋₆, un hydrogène, un hydroxyle ou un halogène, dans la fabrication d'un médicament pour le traitement de la dyskinésie.

2. L'utilisation selon la revendication 1, où R est sélectionné parmi les groupes suivants:

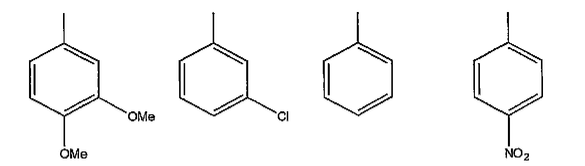

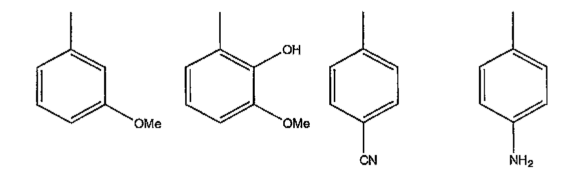

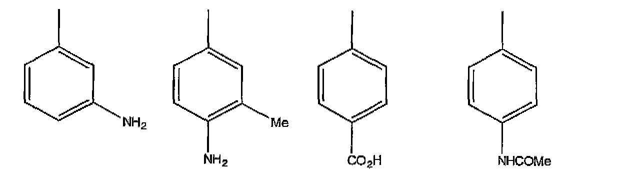

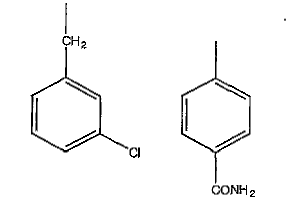

3. L'utilisation selon la revendication 1 ou 2, où lorsque R¹ est un groupe alkyle, c'est un alkyle C₂ (éthyle).

4. L'utilisation selon la revendication 1, 2 ou 3, où lorsque R² est un groupe alcoxy, c'est un alcoxy C₁ (méthoxy).

5. L'utilisation selon l'une quelconque des revendications précédentes, où lorsque R³ est un groupe alcoxy, c'est un alcoxy C₁ (méthoxy),

6. L'utilisation selon l'une quelconque des revendications précédentes, où le composé de la formule I est sélectionné parmi le groupe comprenant le tofisopam, le girisopam et le nerisopam (illustrés ci-dessous):

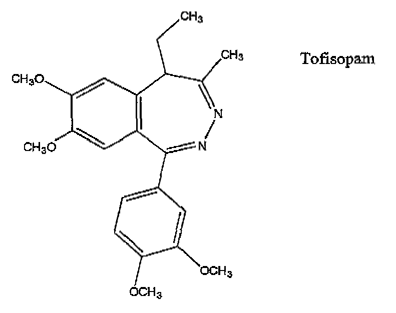

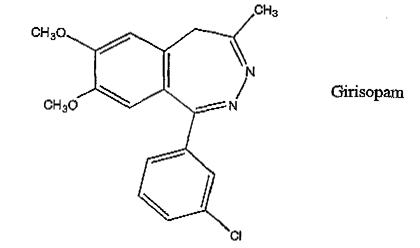

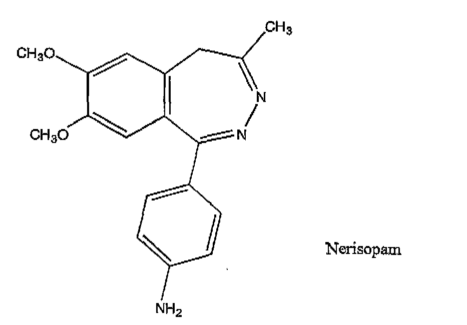

7. L'utilisation selon la revendication 6, où le composé de la Formule 1 est du tofisopam.

8. L'utilisation selon l'une quelconque des revendications 1-7, pour le traitement de la dyskinésie qui survient comme un effet secondaire d'un agent thérapeutique.

9. L'utilisation selon la revendication 8 pour le traitement de la dyskinésie associée à des agents utilisés dans le traitement de troubles de la motricité.

10. L'utilisation selon les revendications 8 ou 9, où l'agent est utilisé pour traiter le parkinsonisme.

11. L'utilisation selon la revendication 10, où l'agent est un précurseur de la dopamine.

12. L'utilisation selon la revendication 10, où l'agent est un agoniste des récepteurs de la dopamine.

13. L'utilisation selon la revendication 10, où l'agent est de la L-DOPA.

14. L'utilisation selon la revendication 10, où l'agent est l'un d'entre le chloro-APB, l'apomorphine, le ropinirole, le pramipexole, la cabergoline, la bromocriptine, le lisuride ou le pergolide.

15. L'utilisation selon la revendication 8, où l'agent est utilisé pour traiter la schizophrénie ou une autre psychose.

16. L'utilisation selon la revendication 15, où l'agent est un neuroleptique.

17. L'utilisation selon la revendication 15, où l'agent possède des propriétés d'antagoniste des récepteurs de la dopamine.

18. L'utilisation selon la revendication 15, où l'agent est de l'halopéridol, de la clozapine, de la fluphénazine ou du sulpiride.

19. L'utilisation selon l'une quelconque des revendications 1-7 pour le traitement d'un trouble hypercinétique associé au syndrome de la Tourette et à ADHD.

20. L'utilisation selon l'une quelconque des revendications précédentes pour un traitement prophylactique.
